# EUROPÄISCHE PATENTANMELDUNG

(11) **EP 1 854 366 A1**
(43) Veröffentlichungstag der Anmeldung: **14.11.2007**
(21) Anmeldenummer: 07009123.6
(22) Anmeldetag: 07.05.2007
(51) Int. Cl.: A23K 1/00, A23K 1/16, A23L 1/0532, A23L 1/29, A23L 1/30, A23L 1/337

(54) **Präparat zur Körperbehandlung**

(30) Priorität: 09.05.2006 DE 102006021478
(71) Anmelder: Tilco Biochemie GmbH, 23858 Reinfeld (DE)
(72) Erfinder: Scharafat, Iradj, 23843 Travenbrück (DE)
(74) Vertreter: Klickow, Hans-Henning

(57) **Zusammenfassung**

Das Präparat dient zur Körperbehandlung und weist eine erste Komponente auf Basis von Algenmaterial und eine mit der ersten Komponente vermischte zweite Komponente auf. Die erste Komponente besteht aus aufgeschlossenem Algenmaterial.

## Beschreibung

Die Erfindung betrifft ein Präparat zur Körperbehandlung, das eine erste Komponente auf Basis von Algenmaterial und eine mit der ersten Komponente vermischte zweite Komponente aufweist.

Derartige Präparate sind bereits sowohl für innere als auch für äußere Anwendungen bereits bekannt geworden. Äußere Anwendungen können in Form sogenannter Algenpackungen im Kosmetikbereich erfolgen, innere Anwendungen sind bereits zur Unterstützung einer Ausscheidung von schädlichen Stoffen aus dem Körper zur Anwendung gekommen. Insgesamt konnten bei den bekannten Verwendungen zwar Wirksamkeiten nachgewiesen werden, die Wirksamkeitsintensität blieb in der Regel aber stark hinter den Erwartungen zurück.

Bekannte derartige Präparate werden einzeln und/oder gemischt eingesetzt, um die Ausscheidung von Schwermetallen aus Nahrungsmitteln durch den Darm zu verbessern. Die bekannten Präparate weisen in der Regel eine spezifische Wirkung auf und es können die Anforderungen an eine Wirksamkeit gegen Schwermetallbelastungen nicht erfüllt werden.

Aufgabe der vorliegenden Erfindung ist es, ein Präparat der einleitend genannten Art derart zu verbessern, daß eine verbesserte Wirksamkeit bereitgestellt wird.

Diese Aufgabe wird erfindungsgemäß dadurch gelöst, daß die erste Komponente aus aufgeschlossenem Algenmaterial besteht.

Durch die Verwendung von aufgeschlossenem Algenmaterial wird eine signifikante Wirksamkeitserhöhung erreicht. Durch das Aufschließen werden die Wirkstoffe mit einer stark erhöhten Zugänglichkeit für den Körper versehen und die wirksamen Oberflächen des Algenmaterials werden erheblich vergrößert. Zum Aufschließen des Algenmaterials können unterschiedliche Verfahren verwendet werden, beispielsweise eine Behandlung mit anorganischen Salzen in einer rotierenden Trommel.

Die obigen Komponentenkombinationen können zusätzlich mit bekannten und in der Praxis angewandten Nahrungs- und Futtermitteln gemischt werden, um eine breitere und/oder längere Wirkung zu erzielen.

Die Präparate können als feste und/oder flüssige Mittel als Getränke oder als Granulat formuliert in Essen oder Futter verabreicht werden. Die Präparate können darüber hinaus auch als Paste eingenommen und realisiert sein.

Die Erfindung betrifft insbesondere Präparate, die Schwermetalle, wie Pb, Cd, u.a. aus belasteten Nahrungsmitteln durch Sorption an aufgeschlossenen Algen (vor allem Braunalgen) (aufgeschlossen mit Alkali- und Erdalkalimetallen, wie z.B. K, Na, Ca, Mg, NH4) einer Ausscheidung durch den Darm zuführen. Die Präparate sind mindestens aus einer ersten Komponente und einer zur ersten Komponente unterschiedlichen zweiten Komponente zusammengesetzt, bei denen die zweite Komponente organische oder anorganische Stoffe enthält, die in Nahrungs- und Futtermitteln aus der Gruppe der Mineralstoffe, Tonminerale, Zusatzstoffe, Nahrungsmittel (pflanzliche sowie tierische) und Nahrungsergänzungsmittel (wie Algenmehl) oder Formulierungsstoffe (wie Maisliesch) realisiert sind.

Die erste Komponente enthält organische Verbindungen, die mindestens zu einem Teil aus mindestens einer Aminosäure sowie zu einem Teil aus mindestens einem Algenmehl-Extrakt, nicht aufgeschlossen und/oder aufgeschlossenen Algenmehlen (mit Natriumoxid, -hydroxid, Carbonate oder und andere Salze oder Kaliumoxid, -hydroxid, -carbonate oder und anderen Salzen bzw. Alkalimetallen behandelt und aufgeschlossen), Schwefel, Schwefelsäure, Schwefelsalze, Eisensulfat, Eisenchlorid, Eisencarbonat, Kalzium, Kalziumoxid, -hydroxid, - carbonat, -nitrat, -phosphat, bestehen.

Als Beispiel sind folgende Verbindungen und Salze zu erwähnen:
Als Komponente des Präparates können flüssige oder feste natürliche oder synthetische organische oder anorganische Verbindungen und Mischungen verwendet werden, die in Nahrungs-, Futter- und Nahrungsergänzungsmitteln von Menschen, Tieren und Fischen, eine Senkung der Schwermetallbelastung und eine Verbesserung der Gesundheit von Menschen, Tiere und Fischen bewirken.

Verwendbar sind beispielsweise:
a) Algenmaterialien wie zum Beispiel frische Algen oder Algenteile, Algenmehle aus verschiedenen Arten und verschiedener Herkunft, insbesondere Braunalgen Laminaria, Ascophylum u.a. sowie Rotalgen in Rohform oder verarbeitet.
b) Algenextrakte und Pasten in allen möglichen Präsentationen
c) Alginate-, Pektinate-, und andere Polysaccharidsalze wie Na, Ca, Cu, K, Mg, Mn, Fe, NH4, usw.
d) Aminosäuren einzeln oder in Mischungen, aus tierischer, pflanzlicher oder synthetischer Herkunft.
e) Zucker und Polysaccharide wie Melasse oder zuckerhaltige Extrakte und Stoffe.
f) Produkte aus Pflanzenmaterial, Sud, Brei, Extrakte etc.
g) Organische und anorganische Materialien wie Getreide, Hackfrüchte, Kartoffeln, Mais, Obst und Gemüse, Fleisch, Fisch, Tonmineralien, Sande, Silizium, Kolloidbildner, slow-release Formulierungshilfsstoffe, synthetische organische und anorganische Substanzen.
h) zugelassene Nahrungs- und Futterergänzungsmittel.
i) Schokolade, Maisliesch u.a.

Als zweite Komponente können Säuren, Laugen und ihre organischen und anorganischen Salze, Schwefel, Schwefelsäure, Schwefligesäure, Phosphor, Phosphorsäure, Phosphorige Säure, Salpetersäure, Salzsäure, Chlor, Kalium, Kalilauge, Kaliumoxid, -hydroxid, Natrium , Natriumoxid, -hydroxid, Natronlauge, Kalzium, Kalziumlauge, Ammoniak, Amoniumlauge verwendet werden, die in Nahrungsmittel eingesetzt werden.

Verwendbar sind beispielsweise als zweite Komponente:

| | | |
|---|---|---|
| a) | Phosphorsäure | H3PO4 |
| aa) | Calziumphosphat | Ca3(PO4)2 |
| ab) | Calziummonophosphat | CaHPO4 |
| ac) | Calziumdiphosphat | Ca(H2PO4)2 |
| ad) | Trippelkalziumphosphat | |
| af) | Kaliumphosphat | K3PO4 |
| ag) | Monokaliumphosphat | KH2PO4 |
| ah) | Dikaliumphosphat | K2HPO4 |
| ai) | Tetrakaliumphosphat | K4P2O7 |
| aj) | Kaliumpolyphosphat | |
| ak) | Natriumphosphat | Na3PO4, |
| al) | Mononatriumphosphat | NaH2PO4 |
| am) | Dinatriumphosphat | Na2HPO4 |
| an) | Tetranatriumphosphat | Na4P2O7 |
| ao) | Natriumpolyphosphat | |
| b) | Phosphorige Säure | H3PO3 |
| ba) | Phosphorige Säure und die Salzen (siehe Patent-Nr. EP 1 298 998 B1) | |
| c) | Schwefel | S |
| ca) | Schwefelsäure | H2SO4 |
| cb) | Sulfate | |
| cd) | Sulfite, Sulfide und Thiosulfate | K2S2O3, NH4)2S2O3, NH4) 2S, K2S, H2S |
| e) | Salpetersäure | HNO3 |
| ea) | Kaliumnitrat | KNO3 |
| eb) | Natriumnitrat | NaNO3 |
| ec) | Kalziumnitrat | CaNO3)2 |
| ed) | Amoniumnitrat | NH4NO3 |
| ef) | Amoniumsulfat | NH4) 2SO4 |
| eg) | Amoniumcarbonat | NH4) 2CO3 |
| ef) | Amoniumphosphat | NH4) 3PO4 |
| ei) | Monoamoniumphosphat | NH4H2PO4 |
| ej) | Diamoniumphosphat | NH4) 2HPO4 |
| | | |
| f) | Magnesiumoxid | MgO |
| fa) | Magnesiumhydroxid | Mg(OH)2 |
| fb) | Magnesiumsulfat | MgSO4 |
| fc) | Magnesiumnitrat | MgNO3)2 |
| fd) | Magnesiumcarbonat | MgCO3 |
| ff) | Magnesiumphosphat | MgHPO4 |
| | | |
| g) | Kalziumoxid | CaO |
| ga) | Kalziumhydroxid | Ca(OH)2 |
| gb) | Kalzimcarbonat | CaCO3 |
| gc) | Kalziumnitrat | CaNO3 |
| gd) | Kalziumsulfat | CaSO4 |
| ge) | Kalziumphosphat | Ca) 3PO4) 2, CaHPO4, Ca2H2P04 |

Die Wirksamkeit ergibt sich aus den nachfolgend erläuterten Versuchen. In den beigefügten Tabellen sind charakteristische Daten zu den nachfolgenden Beispielen zusammengefaßt.

Es zeigen im einzelnen:

**Tabelle 1**

| Ergebnisse Blei (Pb) Gehalt im Blut der Kindern auf Kolahalbinsel (Russland) |
|---|
| Aufgeschlossenen Algenmehl mit Schokolade vermischt mit Milch getrunken |

**Tabelle 2**

| Ergebnisse Blei (Pb) Gehalt im Leber und Muskeln der Masthähnchen |
|---|
| aufgeschlossene Algenmehl mit vermischt mit Futter getrunken |

**Tabelle 3:**

| Ergebnisse Cadmium (Cd) Gehalt im Leber und Muskeln der Masthähnchen |
|---|
| aufgeschlossene Algenmehl mit vermischt mit Futter getrunken |

### Wirkung der ersten Komponenten

Die Wirkung der Algenmehle, auf die Verbesserung der Gesundheit der Mensch und Tieren ist bekannt.

### Wirkung der zweiten Komponenten

Die Wirkung von Säuren und Salzen von Phosphor, Stickstoff, Kalium, Kalzium, Magnesium, Schwefel, Eisen, und Spurennährelementen auf die Tieren und Menschen ist als Futter- und Nahrungsmittel ist ebenfalls bekannt.

Wirkung der aufgeschlossenen Algenmehle einzeln und in der Kombination gemäß Tabelle 1, 2 und 3

Aufgeschlossene und nicht aufgeschlossene Algen mit und ohne Zusatzstoffe, wie beispielsweise Salze, Säuren und oder Laugen des Phosphor, Kalium, Stickstoff, Magnesium, Eisen, Schwefel, haben gesundheitsfördernde Eigenschaften auf die Tiere und Menschen. Die Mischungen aus oben genannten Verbindungen mit aufgeschlossenen Algenmehlen führen zu einer Sorption der Schwermetalle und einer Ausscheidung durch den Darm der Menschen und Tiere. Die Wirksamkeit übersteigt um ein Mehrfaches die Wirkung der einzelnen Stoffe und die Komponenten sind wirksamer als Algenmehl (und oder entsprechende Extrakte). Die Schwermetallaufnahme aus dem Nahrungsmittel wird deutlich verringert.

### Aufwandmenge:

Die Aufwandmenge der oben genannten Produkte, ist abhängig von Fisch-, Tier- und Mensch-Größe und Gewicht.

### Resultate:

Beispiel 1: Die Senkung der Pb-Gehalte im Blut durch Einnahme von aufgeschlossenem Algenmehl bei den Schulkindern ist eindeutig.
Beispiel 2: Die Senkung der Pb-Gehalte in Leber und Muskeln der Masthähnchen durch aufgeschlossene Alginate im Futter im Vergleich zu Kontrolle ist klar ersichtlich.
Beispiel 3: Die Senkung der Cd-Gehalte in Leber und Muskeln der Masthähnchen durch aufgeschl. Alginate im Futter im Vergleich zur Kontrolle ist ebenfalls festzustellen. Während die Senkung durch Algenmehl nicht ausreichend verbessert wird, zeigt das aufgeschlossene Algenmehl eine deutliche Wirkung, da die Sorption durch den Aufschluss der Alginate besser reguliert wird.

Die Produkte können als Granulate, Kapseln, Pillen, Pellets oder als flüssige Mittel oder als Paste formuliert werden.

Sie können auch mit Hilfsmitteln formuliert werden, die einen slow-release- Effekt bewirken.

Im Hinblick auf die ersten Komponenten des Präparates erwiesen sich insbesondere Mischungen aus Algenextrakten mit Algenmehlen oder organischen sowie mineralischen Stoffen, die auch der Ernährung oder Fütterung dienen, als vorteilhaft.

Als organische Materialien sind insbesondere pflanzliche oder und tierische Materialien gemeint.

Bei der Verwendung von Salzen als zweite Komponente des Präparates kann über die jeweilige chemische Zusammensetzung des Salzes die Löslichkeit sowie die Wirkungsgeschwindigkeit beeinflusst werden. Eine langfristige Wirkung wird durch Zusatz von Natrium, Kalium, Ammonium, Kalzium oder deren Säuren und Laugen unterstützt. Spurennährelemente wie Kupfer, Zink, Mangan, Molybdän usw. sind zur Sicherung der Ernährung der Tiere, der Fische und der Menschen vorteilhaft. Bewährt haben sich alle Phosphat-Verbindungen und Sulfite wie in den Kombinationen mit Algen-Extrakten, aufgeschlossenen und nicht aufgeschlossenen Algen sowie Aminosäuren und anderen organischen Stoffen.

Im Hinblick auf die Zusammensetzung der Präparate soll noch einmal darauf hingewiesen werden, dass insbesondere ein Zusatz von Polysacchariden in aufgeschlossenen Algen zu einer gezielten Sorptionsfähigkeit, zum Schutz der Gesundheit der Tiere, Fische und Menschen führt und die Ausscheidung der Schwermetalle durch den Darm verbessert. Darüber hinaus verstärken Säuren, Laugen und Salze, die nur in dieser Zusammensetzung mit aufgeschlossenen Algen formuliert oder granuliert sind, die Tierverträglichkeit.

Die Verwendung von aufgeschlossenem Algenmaterial erweist sich gegenüber einer Verwendung von technischen Alginaten als vorteilhaft, da andere Herstellungsverfahren zur Anwendung kommen, die hier bei der Herstellung von Algenprodukten weniger aggressiv sind als bei der Herstellung von technischen Alginate. Die natürlichen Produkteigenschaften werden durch das Verfahren nicht beeinträchtigt.

**Tabelle 1**

| Ergebnisse Blei (Pb) Gehalt im Blut der Kindern auf Kolahalbinsel (Russland) | | | | | | |
|---|---|---|---|---|---|---|
| Aufgeschlossenen Algenmehl mit Schokolade vermischt mit Milch getrunken | | | | | | |
| | | | | | | |

| **Pb-Gehalte mg Pb/ 100 g Blut (Alter 11- 15)** | | | | | | |
|---|---|---|---|---|---|---|
| | | | | | | |

| Kinder | Kontrolle beim Start 9 Kinder | 0,5 g Kind/Tag aufgeschlossen Algen/Tag 75 Tage | 0,5 g aufgeschlossen Algen/Tag 150 Tage | Differenz bei 75 Tagen Pb-Senkung | Differenz bei 150 Tagen | normal Werte |
|---|---|---|---|---|---|---|
| 1 | 51 | 22,2 | | 28,8 | | 10 |
| 2 | 50 | 15,4 | 14,5 | 34,6 | 35,5 | 10 |
| 3 | 50 | 9,9 | 16,5 | 40,1 | 33,5 | 10 |
| 4 | 45,6 | 14,8 | | 31 | | 10 |
| 5 | 44 | 20 | 5 | 24 | 39 | 10 |
| 6 | 38 | 25 | 8 | 13 | 30 | 10 |
| 7 | 33 | 16 | 5 | 17 | 28 | 10 |
| 8 | 26 | 5,3 | 5 | 20,7 | 21 | 10 |
| 9 | 12,4 | 13,3 | 5 | + 0,6 | 7,4 | 10 |

**Tabelle 2**

| Ergebnisse Blei (Pb) Gehalt im Leber und Muskeln der Masthähnchen | | | | |
|---|---|---|---|---|
| aufgeschlossene Algenmehl mit vermischt mit Futter getrunken | | | | |
| | | | | |

| **Pb-Gehalte an Pb in ppm in Leber und Muskeln** | | | | |
|---|---|---|---|---|
| | | | | |

| Masthänchen Organe, nach eine Fütterungsperiode | Kontrolle | Aufgeschl. Algenmehl 200 ppm in Futter | Aufgeschl. Algenmehl 3000 ppm in Futter | Nicht aufgeschl. Algenmehl 3000 ppm in Futter |
|---|---|---|---|---|
| Leber | 1 | 0,5 | 0,2 | 0,7 |
| Muskeln | 2,2 | 0,3 | 0,3 | 1,5 |

**Tabelle 3**

| Ergebnisse Cadmium (Cd) Gehalt im Leber und Muskeln der Masthähnchen | | | | |
|---|---|---|---|---|
| aufgeschlossene Algenmehl mit vermischt mit Futter getrunken | | | | |
| | | | | |

| **Pb-Gehalte an Cd in ppm in Leber und Muskeln** | | | | |
|---|---|---|---|---|
| | | | | |

| Masthänchen Organe, nach eine Fütterungsperiode | Kontrolle | Aufgeschl. Algenmehl 200 ppm in Futter | Aufgeschl. Algenmehl 3000 ppm in Futter | Nicht aufgeschl. Algenmehl 3000 ppm in Futter |
|---|---|---|---|---|
| Leber | 0,14 | 0,02 | 0,04 | 0,09 |
| Muskeln | 0,05 | 0,03 | 0,01 | 0,03 |

Die Wirksamkeit des verwendeten Algenmaterials kann durch den Vorgang eines Aufschließens wesentlich verbessert werden. Das Aufschließen des Algenmaterials, beispielsweise in Form von Algenmehl, Algenextrakten und/oder Algenpasten, erfolgt durch einen Kontakt des Algenmaterials mit einer Aufschlußsubstanz. Eine derartige Aufschlußsubstanz kann beispielsweise aus Natriumoxid, -hydroxid, carbonat und/oder anderen Salzen oder Kaliumoxid,-hydroxid, - carbonat und/oder anderen Salzen bzw. Alkalimetallen bestehen.

Vorzugsweise erfolgt ein derartiges Aufschließen in einem feuchten Zustand des Algenmaterials nach Wasserzusatz. Bevorzugt erfolgt das Aufschließen mit Verbindungen von Alkalimetallen oder Erdalkalimetallen, insbesondere von Kalium, Natrium, Ammonium und/oder Magnesium. Als Verbindungen kommen insbesondere Oxide, Hydroxide oder Carbonate in Frage. Als Ergebnis des Aufschließens wird vorzugsweise ein granuliertes Produkt erzeugt.

Der Vorgang des Aufschließens kann beispielsweise in Rührkesseln oder in rotierenden Trommeln erfolgen. Gedacht ist beispielsweise an die Verwendung von rotierenden Trommeln mit im wesentlichen horizontal oder schräg verlaufender Drehachse. Der Prozeß des Aufschließens wird vorzugsweise schonend mit mäßiger mechanischer Beanspruchung des Algenmaterials durchgeführt. In Abhängigkeit vom jeweiligen Aufschlußergebnis kann eine leichte Temperierung des Algenmaterials und somit eine Wärmezufuhr von Vorteil sein.

## Patentansprüche

1. Präparat zur Körperbehandlung, das eine erste Komponente auf Basis von Algenmaterial und eine mit der ersten Komponente zweite Komponente aufweist, **dadurch gekennzeichnet, daß** die erste Komponente aus aufgeschlossenem Algenmaterial besteht.

2. Präparat nach Anspruch 1, **dadurch gekennzeichnet, daß** eine Ausbildung für innere Anwendungen realisiert ist.

3. Präparat nach Anspruch 1, **dadurch gekennzeichnet, daß** eine Ausbildung für äußere Anwendungen realisiert ist.

4. Präparat nach einem der Ansprüche 1 bis 3, **dadurch gekennzeichnet, daß** die zweite Komponente als Wirkstoffverstärker ausgebildet ist.

5. Präparat nach einem der Ansprüche 1 bis 4, **dadurch gekennzeichnet, daß** die zweite Komponente eine große Oberfläche aufweist.

6. Präparat nach einem der Ansprüche 1 bis 5, **dadurch gekennzeichnet, daß** eine Ausbildung als Granulat realisiert ist.

7. Präparat nach einem der Ansprüche 1 bis 5, **dadurch gekennzeichnet, daß** eine Ausbildung als Paste realisiert ist.

8. Präparat nach einem der Ansprüche 1 bis 5, **dadurch gekennzeichnet, daß** eine Ausbildung als Flüssigkeit realisiert ist.

9. Präparat nach einem der Ansprüche 1 bis 8, **dadurch gekennzeichnet, daß** das Algenmaterial mindestens zum Teil aus Braunalgen besteht.

10. Präparat nach einem der Ansprüche 1 bis 8, **dadurch gekennzeichnet, daß** das Algenmaterial mindestens zum Teil aus Rotalgen besteht.

11. Präparat nach einem der Ansprüche 1 bis 10, **dadurch gekennzeichnet, daß** die erste Komponente anorganische Komponente oder Komponenten enthält.

12. Präparat nach einem der Ansprüche 1 bis 11, **dadurch gekennzeichnet, daß** die erste Komponente ein Produkt aus Landpflanzen enthält.

13. Präparat nach einem der Ansprüche 1 bis 12, **dadurch gekennzeichnet, daß** ein Produkt aus der Gruppe der Extrakte, Aufschlüsse, Brei, Säfte oder Mehle enthalten ist.

14. Präparat nach einem der Ansprüche 1 bis 13, **dadurch gekennzeichnet, daß** die erste Komponente mindestens ein Polysaccharid enthält.

15. Präparat nach einem der Ansprüche 1 bis 14, **dadurch gekennzeichnet, daß** das Polysaccharid aus der Gruppe Melasse, Pektine, Zucker sowie Salze realisiert ist.

16. Präparat nach einem der Ansprüche 1 bis 15, **dadurch gekennzeichnet, daß** die erste Komponente ein Futter oder Nahrungsmittel enthält.

17. Präparat nach einem der Ansprüche 1 bis 16, **dadurch gekennzeichnet, daß** die zweite Komponente als ein Produkt aus der Gruppe der Kohlenhydrate, Aminosäuren, Fette u. a. realisiert ist.

18. Präparat nach einem der Ansprüche 1 bis 17, **dadurch gekennzeichnet, daß** die erste Komponente synthetische organische Verbindungen enthält.

19. Präparat nach einem der Ansprüche 1 bis 18, **dadurch gekennzeichnet, daß** die erste Komponente Bestandteile aus Wasserpflanzen enthält.

20. Präparat nach einem der Ansprüche 1 bis 19, **dadurch gekennzeichnet, daß** die erste Komponente Bestandteile aus Meereswasserpflanzen enthält.

21. Präparat nach einem der Ansprüche 1 bis 20, **dadurch gekennzeichnet, daß** die erste Komponente Bestandteile aus Süßwasserpflanzen enthält.

22. Präparat nach einem der Ansprüche 1 bis 21, **dadurch gekennzeichnet, daß** die erste Komponente aus der Gruppe der Algen, Extrakte, Breie, Mehle, Aufschlüsse und Alginate enthält.

23. Präparat nach einem der Ansprüche 1 bis 22 **dadurch gekennzeichnet, daß** die erste Komponente synthetische Verbindung enthält.

24. Präparat nach einem der Ansprüche 1 bis 23, **dadurch gekennzeichnet, daß** die erste Komponente ein Kolloid enthält.

25. Präparat nach einem der Ansprüche 1 bis 24, **dadurch gekennzeichnet, daß** die erste Komponente ein Silikatprodukt enthält.

26. Präparat nach einem der Ansprüche 1 bis 25, **dadurch gekennzeichnet, daß** die erste Komponente Erden enthält.

27. Präparat nach einem der Ansprüche 1 bis 26, **dadurch gekennzeichnet, daß** die erste Komponente Tonmineral enthält.

28. Präparat nach einem der Ansprüche 1 bis 27, **dadurch gekennzeichnet, daß** die erste Komponente Sande enthält.

29. Präparat nach einem der Ansprüche 1 bis 28, **dadurch gekennzeichnet, daß** die erste Komponente ein Vitamin enthält.

30. Präparat nach einem der Ansprüche 1 bis 29, **dadurch gekennzeichnet, daß** die erste Komponente Spurennährelemente enthält.
